# FASCICULE DE BREVET EUROPEEN

(11) **EP 0 663 441 B1**
(45) Date de publication et mention de la délivrance du brevet: **25.09.1996**
(21) Numéro de dépôt: 95200590.8
(22) Date de dépôt: 10.03.1995
(51) Int. Cl.: C12N 1/18, A21D 8/04, A21D 6/00, A21D 15/00

(54) **Levure de boulangerie industrielle inactive au froid**
Industrielle, kaltinaktive Backhefe
Industrial low temperature inactive bakers' yeast

(30) Priorité: 16.03.1994 EP 94104043
(43) Date de publication de la demande: 19.07.1995
(73) Titulaire: SOCIETE DES PRODUITS NESTLE S.A., CH-1800 Vevey (CH)
(72) Inventeur: Baensch, Johannes, Condominium, Singapore 0511 (SG); Gysler, Christof, CH-1807 Blonay (CH); Niederberger, Peter, CH-1066 Epalinges (CH)

(56) Documents cités:
- EP-A- 0 196 233
- EP-A- 0 306 107
- EP-A- 0 388 262
- EP-A- 0 487 878
- EP-A- 0 556 905
- WO-A-93/01724

## Description

La présente invention a pour objet un procédé de construction de souches industrielles de levure de boulangerie présentant une propriété Lti, des souches de levure présentant une propriété Lti, l'utilisation desdites souches dans la préparation d'une pâte de boulangerie, une méthode de préparation d'une pâte de boulangerie réfrigérée comprenant une de ces souches de levure, et une pâte de boulangerie comprenant les souches selon la présente invention.

Il est connu que le goût et la texture d'une pâte de boulangerie qui est levée par des levures sont bien supérieurs à ceux d'une pâte qui est levée par des agents chimiques. On connaît également des articles de boulangerie du commerce tels que des pâtes à petits pains et à croissants, qui sont destinés à être conservés au réfrigérateur avant fermentation et cuisson, et qui comprennent cependant un agent de levage chimique.

C'est pourquoi, EP 0487878 décrit l'utilisation de souches de levures présentant une propriété ou un phénotype Lti, autrement dit une propriété d'être peu actives sous réfrigération mais de survivre à ces températures (Lti est le sigle de l'expression anglaise "low temperature inactive"), comme agent de levage pour la production d'articles de boulangerie à cuire au four après conservation sous réfrigération. Cependant, ces levures sont trop vigoureuses c'est à dire dégagent trop de gaz à des températures élevées de réfrigération, c'est à dire entre 8 et 12°C, voire même 14°C, qui sont d'ailleurs communément observées, parfois pendant des temps prolongés dans les conditions usuelles de stockage industrielles de produits réfrigérés. Cette vigueur peut être en particulier attribuée à la présence dans ces souches d'un allèle MAL actif et non réprimé par une voie catabolique, mais aussi à une sélection insuffisante du caractère Lti de ces souches. Enfin, ces levures ne sont également pas optimisées pour répondre favorablement à toutes les conditions nécessaires à leur production industrielle, telle qu'une bonne capacité à être séchée, ou un rendement de croissance élevé, par exemple.

De la même manière, WO 93/01724 et EP 0556905 décrivent l'utilisation de souches de levure présentant une propriété similaire à celle décrite dans EP 0487878. Cependant, les souches décrites appartiennent à une classe de mutants Lts (Lts est le sigle de l'expression anglaise "low temperature sensitive"; Singh et al., 1974, Genetics 77, 651-659) qui présentent souvent une perte rapide de leur viabilité à des températures de réfrigération. De plus, ces levures sont peu adaptées à une production et à une utilisation industrielle optimale. En effet, il s'agit de levures de laboratoire haploïdes et auxotrophes qui ne sont guère aptes a être cultivées sur des milieux industriels traditionnels. De plus, leur potentiel de croissance et leur productivité sur ces milieux sont probablement faibles en comparaison avec ceux d'une souche de levure de boulangerie industrielle, telle que la souche "Levure de boulangerie bleue" (LBB) (Lesaffre, France), par exemple. De plus, la stabilité génétique de ces souches est probablement également insuffisante pour envisager leur production industrielle. Une partie de la production risquerait en effet de perdre le caractère Lts. Enfin, ces souches ne sont probablement guère à même de survivre à un séchage traditionnel de la même manière que peut survivre à un tel séchage une souche de boulangerie industrielle.

WO 93/01724 décrit également une méthode de préparation d'une pâte de boulangerie emballée et réfrigérée comprenant comme levain des levures Lts. Dans cette méthode, on prépare ladite pâte en mélangeant lesdites levures à au moins de la farine et de l'eau, on introduit ensuite cette pâte dans un récipient que l'on scelle, puis on lève la pâte dans ledit récipient que l'on conserve à une température de réfrigération. On obtient ainsi un récipient sous pression contenant une pâte réfrigérée dans laquelle les levures Lts sont censées être quasiment inactives pendant toute la durée du stockage du récipient à froid.

Cette méthode n'est toutefois pas satisfaisante pour différentes raisons. Premièrement, lors de l'ouverture du récipient on observe un gonflement rapide de la pâte due à sa décompression. Malheureusement, ce gonflement est tel qu'il tend à détruire la texture traditionnelle de la pâte.

De plus, les levures Lts sont probablement susceptibles de dégager beaucoup trop de gaz et d'arômes à des températures élevées de réfrigération, c'est à dire entre 8 et 12°C, voire même 14°C, qui sont d'ailleurs communément observées dans les conditions usuelles de stockage industrielle de produits réfrigérés. Ainsi, au bout d'un temps de stockage de quelques semaines, la pression interne du récipient risque d'être telle qu'elle serait susceptible de le faire exploser. De plus, la concentration d'arômes présents à la fois dans la pâte et l'atmosphère du récipient risque également d'être telle qu'elle serait susceptible de dénaturer les qualités olfactives et organoleptiques traditionnelles de la pâte.

En outre, aux températures basses de réfrigération, c'est à dire jusqu'à 8°C, les levures décrites sont censées ne pas dégager de gaz et donc ne pas dégager d'arômes sous peine de faire monter dramatiquement la pression dans le récipient. Ainsi, ces arômes sont censés être produits principalement pendant la levée de la pâte, suite à quoi peu d'entre eux sont produits. De ce fait, les arômes étant par nature instable, la pâte peut également perdre rapidement ses qualités organoleptiques au cours de son stockage.

Enfin, les levures ont été fortement activées par l'étape de levée de la pâte, et il est problématique d'envisager de les inactiver simplement en soumettant la pâte qui les contient à une température de réfrigération. En effet, les levures ont alors un métabolisme fermentaire très actif.

Il serait par conséquent intéressant de favoriser l'apparition des arômes caractéristiques d'une fermentation par des levures, tout au long de la période de conservation de la pâte, sans toutefois dépasser une certaine concentration. De même, il serait avantageux de pouvoir également s'affranchir des limites qu'impose la pression interne du récipient.

La présente invention a pour but de pallier les inconvénients de l'art antérieur, et de proposer ainsi un procédé de construction de souches industrielles de levure de boulangerie présentant une propriété Lti, des levures Lti et une méthode de préparation d'une pâte de boulangerie réfrigérée comprenant une souche desdites levures Lti.

### Résumé de l'invention

A cet effet, dans le procédé de construction d'une souche industrielle de levure de boulangerie présentant un phénotype Lti, c'est à dire la capacité d'être pratiquement inactive mais de survivre dans une pâte à une température inférieure ou égale à 14°C à laquelle la pâte ne congèle pas, et d'être pratiquement inactive jusqu'à 18°C dans un milieu maltosé, on croise premièrement une souche Saccharomyces cerevisiae haploïde présentant la capacité d'être inactive à une température de 3 à 10°C, de préférence au delà de 10°C, avec une souche Saccharomyces cerevisiae haploïde présentant au moins un allèle MAL actif mais sous répression catabolique, puis dans une deuxième étape on croise les ségrégants, et enfin on sélectionne une souche diploïde prototrophe présentant un phénotype Lti, un phénotype Mal actif mais sous répression catabolique, et un potentiel de croissance en processus fed-batch.

Les levures de boulangerie industrielles selon la présente invention présentent un rendement de croissance dans un procédé fed-batch de 0,1 à 0,5 g de biomasse sèche par g de sucre, une production de CO₂ inférieure à 15 ml/h/kg de pâte jusqu'à 8°C, inférieure à 20 ml/h/kg de pâte jusqu'à 12°C, et en outre inférieure à 10 ml par gr de levure pressée jusqu'à une température de 18°C après 4 jours de culture en milieu maltosé.

Dans le procédé de préparation d'une pâte de boulangerie réfrigérée et emballée, on prépare ladite pâte en mélangeant des levures selon la présente invention à au moins de l'eau et de la farine, puis on emballe cette pâte dans un récipient qui comprend une soupape de sortie de gaz.

La pâte de boulangerie selon la présente invention comprend au moins de l'eau, de la farine et une souche de levure Lti selon la présente invention, et est conservée à une température de réfrigération pendant au moins 1 ou 2 jours.

Ainsi on a pu construire des souches remarquables de levure de boulangerie Lti présentant la propriété d'être inactives mais vivantes dans une pâte aux températures de réfrigération, et même d'être encore pratiquement inactives à des températures supérieures, c'est à dire jusqu'à 18°C.

Ces souches de levure de boulangerie présentent également la propriété d'être plus adaptées à une utilisation industrielle. Ces souches présentent ainsi un rendement de croissance proche, voire égal à celui d'une souche industrielle commercialisée, telle que la levure LBB. Ces souches se prêtent également remarquablement bien à un séchage en vue de leur conservation sans qu'elles perdent leur activité d'une manière significative après réhydratation. Enfin, il faut également noter la stabilité génétique surprenante de ces levures pour leur caractère Lti.

Enfin, grâce à la méthode de préparation de ladite pâte selon la présente invention, on évite tous les inconvénients liés à la pression interne du récipient. On peut utiliser ainsi un matériel d'emballage plus souple. Le goût de la pâte est également amélioré du fait de l'activité résiduelle des levures qui développent des arômes tout au long de la période de conservation, sans toutefois faire lever la pâte, et du fait également de la sortie vers l'extérieur de l'emballage de l'excès d'arômes produits au cours de la période de stockage.

Enfin, au cours de son stockage la pâte se sature en gaz, ce qui permet directement de la cuire car la présence de gaz dans la pâte suffit à la faire gonfler lors de sa cuisson. On évite ainsi une étape préliminaire de levée de la pâte.

Par l'expression "allèle MAL" on entend dans le présent exposé, un gène complexe faisant partie de la famille des gênes MAL codant pour trois fonctions, une α-glucosidase, une perméase et une protéine de régulation. Et, par l'expression "allèle MAL actif mais sous répression catabolique" on entend un allèle MAL dont l'expression de ses fonctions est réprimée par la présence dans le milieu de culture de glucose ou de saccharose (allèle MAL inductible) par exemple, ou un allèle MAL qui exprime ses fonctions mais dont les fonctions elles mêmes sont réprimées par la présence dans le milieu de culture de glucose ou de saccharose (allèle MAL constitutif), par exemple.

Par l'expression "levures Lti" on entend les levures qui ont la propriété ou le phénotype d'être pratiquement inactives mais vivantes dans une pâte à une température égale ou inférieure à 14°C à laquelle la pâte ne congèle pas, et d'être aussi pratiquement inactives jusqu'à 18°C dans un milieu maltosé.

De même, par l'expression "potentiel de croissance", on entend une aptitude à être cultivée avec un bon rendement et une bonne productivité par un processus utilisable industriellement, notamment par le processus de culture traditionnel de la levure de boulangerie connu sous le nom de fed-batch (addition lente et progressive d'une solution de sucre à une suspension sous aération, de manière à éviter la formation d'alcool au cours de la production de biomasse et à maximiser le rendement).

De plus, par le terme "prototrophe", on entend une levure qui se développe sur un milieu de culture comprenant uniquement une source de carbone, d'azote, de phosphate, de souffre, des oligoéléments et des traces de vitamines. Ce milieu peut être appelé "milieu minimum".

En outre, par l'expression "température de réfrigération" on entend toutes les températures inférieures ou égales à 12°C auxquelles la pâte ne congèle pas.

Enfin, dans la suite de la description le terme "pâte" ou "pâte de boulangerie" désigne une pâte de boulangerie traditionnelle telle qu'une pâte à pizza, une pâte à pain ou une pâte à croissant, par exemple. Ces pâtes peuvent comprendre en particulier au moins une quantité appropriée de farine de blé, de sels, d'huile, et d'eau, par exemple.

### Description des dessins

### Figure 1:

Représentation du niveau de production de CO2 en fonction du temps pour 100 gr de pâte comprenant 1% de levure pressée, à 8°C, 12°C, et 30°C, pour les souches NCIMB 40611, 40612, et une levure de boulangerie Hindelbank.

### Figure 2:

Représentation tridimensionnelle du niveau de production de CO2 en milieu maltosé en fonction de la température et de la durée pour les souches NCIMB 40612.

### Figure 3:

Représentation tridimensionnelle du niveau de production de CO2 en milieu maltosé en fonction de la température et de la durée pour la "levure de boulangerie bleue" (LBB, pour comparaison).

### Description détaillée de l'invention

Pour mettre en oeuvre le présent procédé de construction d'une souche de levure présentant une propriété Lti, on croise premièrement une souche Saccharomyces cerevisiae haploïde présentant une propriété Lti avec une souche Saccharomyces cerevisiae haploïde présentant au moins un allèle MAL actif mais sous répression catabolique.

Pour ce faire, on peut choisir une souche Saccharomyces cerevisiae haploïde présentant une bonne propriété Lti, c'est-à-dire la capacité d'être inactive entre 3 et 10°C, de préférence au delà de 10°C, par exemple.

On peut également choisir une souche Saccharomyces cerevisiae présentant au moins un allèle MAL sous répression catabolique qui est inductible, ce qui signifie que l'α-glucosidase n'est pas présente constitutivement dans cette levure cultivée dans un milieu de culture industriel. En effet, ces milieux comprennent comme sucres principalement des sucres responsables de la répression catabolique, par exemple du glucose et/ou du saccharose. Par contre, dans une pâte de boulangerie cette levure synthétisera l'α-glucosidase en vue d'utiliser le maltose de la pâte seulement lorsque les sucres responsables de la répression catabolique auront été épuisés par la levure. On peut ainsi choisir l'allèle MAL2, MAL3 ou MAL6, par exemple.

De plus, l'allèle MAL sous répression catabolique peut être également constitutif, ce qui signifie que l'α-glucosidase est naturellement présente dans la levure tout en étant réprimée par la présence de glucose ou de saccharose du milieu de culture. Dans une pâte de boulangerie cette levure utilisera alors directement le maltose de la pâte seulement lorsque les sucres responsables de la répression catabolique auront été épuisés par la levure. On peut ainsi choisir l'allèle MAL2-8c, par exemple.

Les descendants diploïdes issus du premier croisement peuvent être mis alors à sporuler sur un milieu de sporulation traditionnel, pour obtenir des ségrégants haploïdes. On peut à ce stade, vérifier les caractères Lti et Mal des ségrégants haploïdes, en les cultivant sur un milieu comprenant comme seule source de carbone du maltose, par exemple le milieu YPM-Agar (voir chapitre milieux) pour le caractère Mal, et sur un milieu YPD-Agar à une température de réfrigération de 8°C, pendant au moins 21 jours pour le caractère Lti, par exemple. Après au moins 21 jours, les colonies Lti qui survivent mais qui demeurent relativement actives sont sélectionnées en fonction de leur développement cellulaire visible comparé à celui du parent haploide non-Lti. On choisit de préférence les colonies qui se développent lentement à cette température.

Ensuite, dans une deuxième étape on croise entre eux les ségrégants de sexe opposés sur un milieu approprié. On peut ainsi croiser des ségrégants provenant du même croisement premier, ou croiser des ségrégants provenant d'un croisement premier différent, c'est-à-dire un croisement de deux ségrégants qui ont chacun des parents qui peuvent être différents, ou dont l'un des parents peut être le même. On obtient ainsi un grand nombre de souches de levure diploïde présentant une grande diversité de caractéristiques.

On peut vérifier le caractère prototrophe des levures diploïdes en les cultivant sur milieu minimum, par exemple. On peut également vérifier le phénotype Mal en les cultivant sur le milieu "YPM-Agar, par exemple. Les levures qui ont hérité de leurs parents un ou plusieurs gènes MAL sous répression catabolique vont ainsi utiliser le maltose et être alors révélées par l'indicateur de pH présent dans le milieu. On peut de plus vérifier le caractère diploïde des levures, en les faisant sporuler sur un milieu approprié, par exemple. Enfin, on peut vérifier la propriété Lti des souches diploïdes en les cultivant sur milieu YPD-Agar à une température de réfrigération de 8°C pendant 21 jours, par exemple. Les souches Lti s'y développent alors très lentement.

Enfin, on sélectionne parmi ces souches de levure diploïde, une souche prototrophe présentant un phénotype Lti, un phénotype Mal actif mais sous répression catabolique, et un potentiel de croissance en processus fed-batch.

A cette étape, on peut retenir donc des critères de sélection plus sévères et plus complets que ceux utilisés au cours des étapes précédentes. On peut ainsi sélectionner une propriété Lti particulière en soumettant les souches à un test de production de CO2 dans une pâte de boulangerie, durant plusieurs jours, à des températures comprises entre 3 et 30°C, notamment entre 8 et 14°C, par exemple.

On peut également sélectionner ladite souche diploïde en fonction d'un potentiel de croissance, c'est à dire d'un taux de croissance exponentielle en processus fed-batch, en la développant dans un milieu de culture qui demande chez les levures une métabolisation respiratoire de la ou des source(s) de carbone. On peut ainsi sélectionner des souches ayant un taux de croissance exponentielle égal ou supérieur à 50% de celui d'une souche de levure industrielle référencée qui n'a pas une propriété Lti, comme par exemple la souche LBB, mais de préférence les souches ayant ledit taux égal ou supérieur à 65%, et en particulier les souches ayant ledit taux proche ou égal à 100%.

En mettant en oeuvre le procédé de construction selon la présente invention, on peut donc construire des souches de levure qui présentent une propriété Lti remarquable. En effet, la répression du gène MAL provoque un retard d'activité des levures présentes dans une pâte qui est soumise à une température à laquelle ces levures redeviennent potentiellement actives. Ce retard s'explique en effet par le fait que les levures doivent d'abord utiliser les sucres responsables de la répression catabolique avant de pouvoir utiliser le maltose, et que ces sucres ne sont pas présents en quantité suffisante pour assurer une activité fermentaire optimale des levures.

En outre, ce retard est encore plus important lorsque les levures Lti possèdent un allèle MAL inductible mais sous répression catabolique, car en effet la levure doit en plus synthétiser les fonctions capables d'utiliser le maltose après qu'elle ait utilisé les sucres responsables de la répression catabolique. Ces levures Lti sont de ce fait encore moins sensibles à des variations de températures au-dessus des températures de réfrigération.

Ces levures peuvent donc être utilisées dans la production d'articles de boulangerie à cuire au four après conservation sous réfrigération.

Les levures de boulangerie industrielles selon la présentent invention sont susceptibles d'être obtenues par le présent procédé de construction. Ces souches de boulangerie industrielle Saccharomyces cerevisiae présentent ainsi un rendement de croissance en processus fed-batch de 0,1 à 0,5 g de biomasse sèche par g de sucre(s), une production de CO2 inférieure à 15 ml/h/kg de pâte jusqu'à une température de 8°C, inférieure à 20 ml/h/kg de pâte jusqu'à 12°C, et une production de CO2 inférieure à 10 ml par gr de levure pressée jusqu'à 18°C, après 4 jours de culture en milieu maltosé.

De préférence, les levures selon la présente invention présentent une production de CO2 inférieure à 7 ml/h/kg de pâte jusqu'à une température de 8°C, notamment 0,1 à 7 ml/h/kg, et inférieure à 12 ml/h/kg de pâte jusqu'à 12°C, notamment 0,1 à 12 ml/h/kg.

On peut également sélectionner des souches de levures qui présentent une production de CO2 inférieure à 3 ml/h/kg de pâte jusqu'à une température de 12°C, par exemple.

De même, on peut sélectionner des souches de levures qui présentent un rendement de croissance en processus fed-batch égal ou supérieur à 0,5 g de biomasse sèche par g de sucre(s), par exemple.

Parmi diverses souches de Saccharomyces cerevisiae ainsi obtenues, on en a déposé deux à titre d'exemples, selon le Traité de Budapest, le 28.01.94 , à la National Collection of Industrial and Marine Bacteria Ltd. (NCIMB), P.O.Box 31, 135 Abbey Road, ABERDEEN AB9 8DG, Ecosse (Royaume uni) où elles ont reçu les numéros NCIMB 40611 et 40612.

La souche NCIMB 40611 présente en outre les caractéristiques suivantes.
Morphologie: cellules elliptiques d'une taille relativement homogène.
Fermentation: levures capables de fermenter le saccharose, le glucose et le maltose.

De même, La souche NCIMB 40612 présente les caractéristiques suivantes.
Morphologie: cellules elliptiques d'une taille relativement homogène.
Fermentation: levures capables de fermenter le saccharose, le glucose et le maltose.

Dans le procédé de préparation d'une pâte de boulangerie réfrigérée et emballée selon la présente invention, on prépare une pâte en mélangeant une levure selon la présente invention à au moins de l'eau et de la farine, puis on emballe cette pâte dans un récipient qui comprend une soupape de sortie de gaz.

On peut ainsi mélanger puis emballer la pâte à des températures de réfrigération.

L'atmosphère du récipient peut être également composée d'un gaz inerte tels que de l'azote ou du dioxyde de carbone, seul ou en combinaison. Cette atmosphère peut être crée, par exemple, en aspirant l'oxygène du récipient par le vide puis en insufflant le gaz inerte avant de sceller ledit récipient. Cette atmosphère permet en particulier d'éviter un développement de champignons.

En particulier, on peut également mélanger la levure à la pâte à raison de 0,1 à 1% en matière sèche de levure pressée ou réhydratée.

Ces levures peuvent en outre contenir du tréhalose. Et, on peut ajouter à la pâte 1,2 à 2% de NaCl, afin de diminuer le développement excessif de la flore naturelle de la pâte.

La pâte réfrigérée selon la présente invention peut être préparée et emballée en utilisant la méthode de préparation d'une pâte décrite ci-dessus. Cette pâte présente en outre une texture et un arôme caractéristiques d'une pâte ayant la même composition et qui est levée par des levures de boulangerie traditionnelles, comme la levure LBB. On peut cependant préférer la pâte réfrigérée de la présente invention, lorsque celle-ci est conservée pendant au moins 2 ou 3 jours à des températures usuelles de réfrigération. En effet, après 2 ou 3 jours de conservation de la pâte à des températures de réfrigération, les levures Lti produisent suffisamment de gaz dans la pâte pour que celle-ci gonfle directement lorsqu'elle est cuite. Une étape de levée de la pâte est donc évitée.

Les exemples ci-après sont présentés à titre d'illustration du procédé de construction de souches Lti, du procédé de préparation d'une pâte selon la présente invention, des souches Lti obtenues par le procédé de construction, et de la pâte emballée et réfrigérée obtenue par le présent procédé de préparation. Les pourcentages sont donnés en poids sauf indication contraire.

Ces exemples sont précédés d'une description de divers tests, de la composition de divers milieux, et d'une brève présentation des dessins.

### Tests

### 1. Production de CO2 dans une pâte:

Pour réaliser ce test, on prépare une pâte de boulangerie qui servira de milieu de culture. Cette pâte comprend 60,2% de farine, 29,2% d'eau, 1,4% de NaCl, 7.2% d'huile de cacahouète, et 2% de levures Lti à 15% de matière sèche. Tous les ingrédients sont conservés à 4°C et on prépare la pâte entre 4-8°C.

Pour cela, on prépare premièrement une suspension homogène de levures en mettant quelques grammes d'une suspension de levures Lti à 30% de matière sèche dans un tube Falcon de 50 ml, et en ajoutant une quantité équivalente d'eau de façon à obtenir une suspension à 15% de matière sèche, puis en agitant le tout. Ensuite, on mélange la farine et le sel, puis on ajoute l'huile de cacahouète et l'eau, et enfin on ajoute la suspension de levure Lti. Finalement, on brasse la pâte jusqu'à ce qu'elle ne colle plus, sans toutefois trop la travailler. On obtient alors une pâte lisse. On prélève alors 100 gr de cette pâte que l'on transfère dans un Risograph (RDesign, USA), puis on mesure immédiatement le dégagement de CO2.

Le dégagement de gaz est mesuré premièrement à 8°C toutes les heures pendant 120 heures. Puis on élève la température à 12 °C et on mesure le dégagement de gaz toutes les heures pendant 120 heures. Enfin, on élève la température à 30 °C et on mesure le dégagement de gaz toutes les 10 minutes pendant 6 heures. L'activité des levures est alors déterminée en calculant la pente initiale de dégagement de gaz (ml/h/kg de pâte) pour chacune des températures.

### 2. Potentiel de croissance:

Ce test a pour but de sélectionner des souches de levure qui présentent une capacité de croissance proche de celle de souches de levure industrielle.

Pour ce faire, on cultive les souches de levure dans un milieu qui induit un métabolisme similaire à celui de souches cultivées en fermentation fed-batch. On sait en effet que dans un processus fed-batch traditionnel les levures assimilent les sources de carbone par des voies métaboliques respiratoires, qu'elles n'accumulent pas ou peu d'éthanol et d'acétate, et qu'elles sont capables en plus de les réassimiler très rapidement par des voies respiratoires. On sélectionne ainsi les souches en les cultivant sur un milieu de culture qui demande aux levures une métabolisation respiratoire de la ou des source(s) de carbone.

Pour cela, on cultive les levures en Erlenmeyer (qui comprennent des chicanes qui favorisent l'aération du milieu) dans un milieu YNEA comprenant comme source de carbone de l'éthanol et de l'acétate. Une faible quantité d'extrait de levure est ajoutée dans le but de mimer les milieux usuels industriels, tel qu'un milieu comprenant de la mélasse. De plus, on tamponne le milieu à pH 4 avec du succinate et de l'acide hydrochlorique pour favoriser la présence de l'acétate sous sa forme acide.

Dans ce test, on pré-cultive les levures que l'on teste dans 5 ml de milieu YPD à 30°C, sous agitation à environ 190 rotations par minute (rpm) pendant 12 heures à 30°C. Puis, on inocule 100 ml de milieu YNE (contenu dans un Erlenmeyer de 500 ml comprenant les chicanes) avec 1 ml de la pré-culture, et on incube 24 heures à 30°C sous agitation (190 rpm). Enfin, on inocule 100 ml de milieu YNEA (contenu dans un Erlenmeyer de 500 ml comprenant les chicanes) avec la culture précédente en une quantité telle que l'on atteint une DO₆₀₀ proche de 0,1, et l'on incube à 30°C sous agitation pendant 10 heures en mesurant la DO₆₀₀ toutes les 2 heures.

On sélectionne alors les souches présentant le meilleur taux de croissance exponentielle. On choisit ainsi de préférence les souches qui présentent un taux de croissance exponentielle supérieur ou égal à 50% de celui de la souche LBB, en particulier supérieur ou égal à 65%, et notamment proche ou égal à 100%.

### 3. Rendement en culture fed-batch:

On réalise une culture des levures sélectionnées dans des conditions industrielles de fermentation fed-batch.

Pour cela, on cultive premièrement les levures à 30°C sous agitation dans plusieurs récipients contenant chacun 400 ml de milieu YPD. Puis on inocule stérilement avec 3 litres des cultures précédentes un fermenteur de 30 litres (Chemap, Suisse) contenant 8 litres de milieu "pré-mélasse" (voir chapitre "milieux"). Enfin, on réalise une fermentation selon le processus fed-batch traditionnel en incubant à 30°C sous agitation (250 à 450 rpm) et aération croissantes (0,02 à 0,8 m³/h), pendant 24 heures, en maintenant le pH à 4,5 par ajout de quantités adéquates de NH₄OH, en contrôlant la mousse produite par ajout de quantités croissantes d'agent anti-moussant tel que du Contraspum 210 (1,5% poids/volume de milieu; Binggeli-Chemie, Suisse), et en ajoutant régulièrement une quantité croissante adéquate de milieu "mélasse" jusqu'à un volume final d'environ 22 litres.

Finalement, on transfère stérilement le produit de cette dernière fermentation dans un fermenteur de 300 litres (Chemap, Suisse) et on réalise à nouveau une fermentation fed-batch en incubant à 30°C sous agitation (240 à 350 rpm) et aération croissantes (0,2 à 10 m³/h), pendant 28 heures, en maintenant le pH à 4,5 par ajout de quantités adéquates de NH₄OH, en contrôlant la mousse produite par ajout de quantités croissantes d'agent anti-moussant tel que du Contraspum (5% poids/volume), et en ajoutant régulièrement une quantité croissante adéquate de milieu "mélasse" pendant 26 heures jusqu'à un volume final d'environ 180 litres.

Pendant les deux dernières heures de fermentation, on n'ajoute donc plus de mélasse, on diminue ainsi la quantité de résidus de substrat dans le milieu, de manière à éviter la présence de sucres qui favoriseraient la croissance des levures à une température de réfrigération.

Enfin, on favorise par la même, également, la formation de tréhalose dans les levures. Ce sucre de stockage est relativement important pour accroître la viabilité des levures dans un procédé de séchage traditionnel.

Le tableau suivant illustre la quantité de milieu "mélasse" que l'on ajoute en fonction du temps, pour les fermentations de 30 et 300 litres.

| | Fermentation fed-batch 30 litres | Fermentation fed-batch 300 litres |
|---|---|---|
| temps (h) | milieu mélasse (g/h) | milieu mélasse (g/h) |
| 1 | 62.5 | 600 |
| 2 | 62.5 | 600 |
| 3 | 62.5 | 700 |
| 4 | 62.5 | 800 |
| 5 | 71 | 900 |
| 6 | 79 | 1000 |
| 7 | 87 | 1100 |
| 8 | 95 | 1200 |
| 9 | 102 | 1300 |
| 10 | 110 | 1400 |
| 11 | 118 | 1500 |
| 12 | 133 | 1600 |
| 13 | 147 | 1700 |
| 14 | 162 | 1800 |
| 15 | 177 | 1900 |
| 16 | 191 | 2000 |
| 17 | 206 | 2100 |
| 18 | 220 | 2200 |
| 19 | 235 | 2300 |
| 20 | 249 | 2400 |
| 21 | 264 | 2500 |
| 22 | 279 | 2600 |
| 23 | 293 | 2700 |
| 24 | 308 | 2800 |
| 25 | /// | 2800 |
| 26 | /// | 2800 |
| 27 | /// | 0 |

On refroidit alors la culture du fermenteur de 300 litres jusqu'à 12°C, puis on la centrifuge (centrifugeuse Westfalia) jusqu'à obtenir environ 20% de matière sèche, et puis on resuspend les levures sédimentées dans de l'eau. On re-centrifuge ensuite cette suspension tout en lavant la biomasse durant cette centrifugation avec environ 300-400 litres d'eau stérile pour 100 litres de suspension.

On peut ajouter à ce stade de la vitamine C pour diminuer l'oxydation de la biomasse. La vitamine C présente également l'avantage de réduire le pH à 4,4.

Finalement, on centrifuge la biomasse (centrifugeuse "De Laval") pour ôter des impuretés jusqu'à obtenir 28% de matière sèche, puis on presse ladite biomasse (presse Bücher) jusqu'à obtenir une matière sèche de 34 à 35%. On obtient alors des levures pressées.

On peut alors déterminer le rendement de croissance des levures. Pour cela, on détermine la quantité de biomasse sèche obtenue pour la quantité de sucres ajoutée au cours de la fermentation (principalement du saccharose présent dans la mélasse).

### 4. Stabilité génétique:

La stabilité génétique d'une souche de levure pour un caractère particulier tel que le caractère Lti, est un facteur décisif pour l'industrialisation d'une telle souche.

Pour apprécier cette stabilité on recherche des révertants pour le caractère Lti. Pour cela, on étale environ 10⁸ cellules d'une culture de cellules en processus fed-batch industriel sur 10 boîtes de Petri contenant un milieu YPD, on incube les boîtes à 8°C, puis on détermine les colonies qui se développent à cette température après environ 4 semaines.

### 5. Production de CO2 sous un gradient de température.

On réalise ce test dans un appareil spécialement conçu pour cela qui comprend un bloc à gradient de température présentant des loges à températures variables, par exemple, dans lesquelles on peut introduire l'extrémité inférieure de tubes de fermentation. Ces tubes présentent une extrémité supérieure fermée et graduée, ainsi qu'un ballon d'expansion branché sur le côté. Le CO₂ produit par la levure s'accumule dans l'extrémité supérieure graduée de chaque tube, le milieu de culture déplacé par l'accumulation du gaz pouvant passer dans le ballon d'expansion.

Pour réaliser ce test, on inocule 2 ml d'une culture d'une nuit sur milieu YPD de la souche à tester dans 200 ml d'un premier milieu contenant 0,67% d'une base azotée sans acides aminés, telle que le produit commercialisé par la maison Difco sous la désignation "yeast nitrogen base without amino acids", par exemple, 0,5% d'extrait de levure, 2% de saccharose, 1% de succinate de sodium et de l'acide chlorhydrique concentré pour ajuster le pH à 4,5, dans un flacon de 500 ml. On incube sous agitation durant 24 h à 30° C.

On sépare les cellules par centrifugation sous 6000 gₙ à 20°C durant 5 min et on les met en suspension dans 200 ml d'un deuxième milieu contenant 0,67% d'une base azotée sans acides aminés, 0,3% d'extrait de levure et 0,3% de saccharose, 1% de succinate de sodium et de l'acide chlorhydrique concentré pour ajuster le pH à 4,5, dans un flacon de 500 ml. On incube sous agitation durant 24 h à 30° C.

On sépare les cellules par centrifugation sous 6000 gₙ à 4° C durant 5 min et l'on lave deux fois le culot de cellules de levure obtenu avec 50 ml d'eau distillée.

On suspend les cellules dans 10 ml d'eau distillée et on les transfère dans des tubes de polypropylène de 15 ml gradués et pré-pesés. On les centrifuge sous 3000 gₙ à 4° C durant 10 min. On égoutte les tubes, on pèse les culots de levure et on les met en suspension, à raison de 0,61 g de culot de levure équivalent à 0,5 g de levure pressée présentant une teneur en matière sèche d'environ 27%, par ml, dans un troisième milieu contenant 0,67% de base azotée sans acides aminés, 2% de maltose, 1% de succinate de sodium et de l'acide chlorhydrique concentré pour ajuster le pH à 4,5. On introduit 0,5 à 3 ml (pour des températures > 10° C) ou 1 à 3 ml (pour des températures < 10° C) dans des tubes de fermentation tels que décrits ci-dessus que l'on a remplis au préalable chacun avec 50 ml dudit troisième milieu, autrement dit du milieu maltosé, et que l'on a refroidis à 4°C.

On incube les tubes de fermentation aux températures désirées dans le bloc à gradient de température décrit ci-dessus. On note la production de CO₂ à intervalles choisis après avoir plongé les tubes quelques secondes dans un bain à agitation sonique pour dégager les bulles de CO₂ retenues dans le milieu liquide.

### Milieux

Les pourcentages des composés sont donnés en poids/volume sauf indication contraires.
- YPM-Agar:: 1% "Difco Bacto Yeast extract".
2% "Difco Bacto Peptone".
2% Maltose.
2% "Difco Bacto Agar".
0,9% volume/volume (v/v) d'une solution de 0,4% (v/v) de Rouge de Bromcresol dissout dans l'éthanol pur.
- YPD:: 2% "Difco Bacto Peptone".
1% "Difco Bacto Yeast extract".
2% Glucose.
- YPD-Agar:: YPD comprenant 2% de "Difco Bacto Peptone"
- YNE:: 0,67% "Difco Yeast Nitrogen Base sans acides aminés".
0,5% "Difco Bacto Yeast extract".
1% Na₂-Succinate.
1,12% (v/v) HCl 5M.
0,7% (v/v) éthanol.
- YNEA:: YNE avec 0,3% (v/v) d'acide acétique glacial.

Pré-mélasse: Le milieu initial de fermentation fed-batch contient le milieu pré-mélasse ayant la composition suivante.
100 kg d'eau déminéralisée
263 g (NH₄)2HPO₄
62 g MgSO₄
8.6 g NaCl
7,82 g CaCl₂
17 g Inositol
0.84 g Ca-Pantothenate
0.006 g Biotine

### Mélasse:

84.85% de mélasse stérile (Aarberg, Suisse).
13.85% d'eau.
1% H2SO4.

On opère la stérilisation de la mélasse de la manière suivante. On induit la germination des spores contaminantes par traitement thermique à 80°C pendant 30 minutes, on laisse germer à température ambiante pendant 20 heures, on stérilise à 125°C pendant 2 minutes, puis après refroidissement on sépare les sédiments par centrifugation (centrifugeuse Westfalia).

### Exemple 1

On part d'une souche haploïde de Saccharomyces cerevisiae présentant une propriété Lti, telle que la souche NCIMB 40613 présentant le génotype MATa, mal3, lts500 que l'on a déposée selon le traité de Budapest le 28.01.94, à la National Collection of Industrial and Marine Bacteria Ltd. (NCIMB), P.O.Box 31, 135 Abbey Road, ABERDEEN AB9 8DG, Ecosse (Royaume uni).

On croise cette souche avec une souche haploïde Saccharomyces cerevisiae comprenant un allèle MAL actif mais sous répression catabolique et inductible, notamment la souche NCIMB 40614 présentant le génotype MATa, trp5, ade7, ade1, MAL6, déposée le 28.01.94 selon le traité de Budapest au même organisme de dépôt que celui cité ci-dessus.

On fait sporuler les levures diploïdes, on isole des spores haploïdes, puis on caractérise leur type sexuel par des moyens traditionnels, leur phénotype Mal par culture sur milieu YPM-Agar, et leur phénotype Lti par culture sur milieu YPD-Agar à 8°C pendant 21 jours. Après 21 jours, les colonies Lti se distinguent par une faible croissance comparée à celle du parent haploid non-Lti, c'est à dire la souche NCIMB 40614.

Environ 25% des ségrégants haploides partagent les phénotypes Lti et Mal, et sont ainsi sélectionnés.

Puis, on croise entre eux les ségrégants de sexe opposé, et on sélectionne alors des souches diploïdes de levures prototrophes. Pour cela, on vérifie le phénotype Mal en les cultivant sur milieu YPM-Agar. Environ 50% des souches diploides partagent les phénotypes Lti et Mal.

On sélectionne aussi une propriété Lti en soumettant les souches au test de production de CO2 dans une pâte de boulangerie décrit ci-dessus. Environ, 100% des souches diploides ont une production de CO2 inférieure à 15 ml/h/kg de pâte jusqu'à 8°C, et inférieure à 20 ml/h/kg de pâte jusqu'à 12°C. En outre, environ 60% des souches diploides ont une production de CO2 inférieure à 7 ml/h/kg de pâte jusqu'à 8°C, et inférieure à 12 ml/h/kg de pâte jusqu'à 12°C.

On sélectionne aussi un potentiel de croissance en processus fed-batch en cultivant les souches selon le test "potentiel de croissance" décrit ci-dessus. Environ 50% des souches diploides ont un taux de croissance exponentielle supérieur à 65% de celui de la souche LBB référencée. Environ 30% des souches diploides ont un taux de croissance exponentielle supérieur à 80% de celui de la souche LBB. Et environ 12% des souches diploides ont un taux de croissance exponentielle supérieur à 90% de celui de la souche LBB.

Parmi diverses souches ainsi obtenues, on a déposé, à titre d'exemple, la souche NCIMB 40611 mentionnée plus haut.

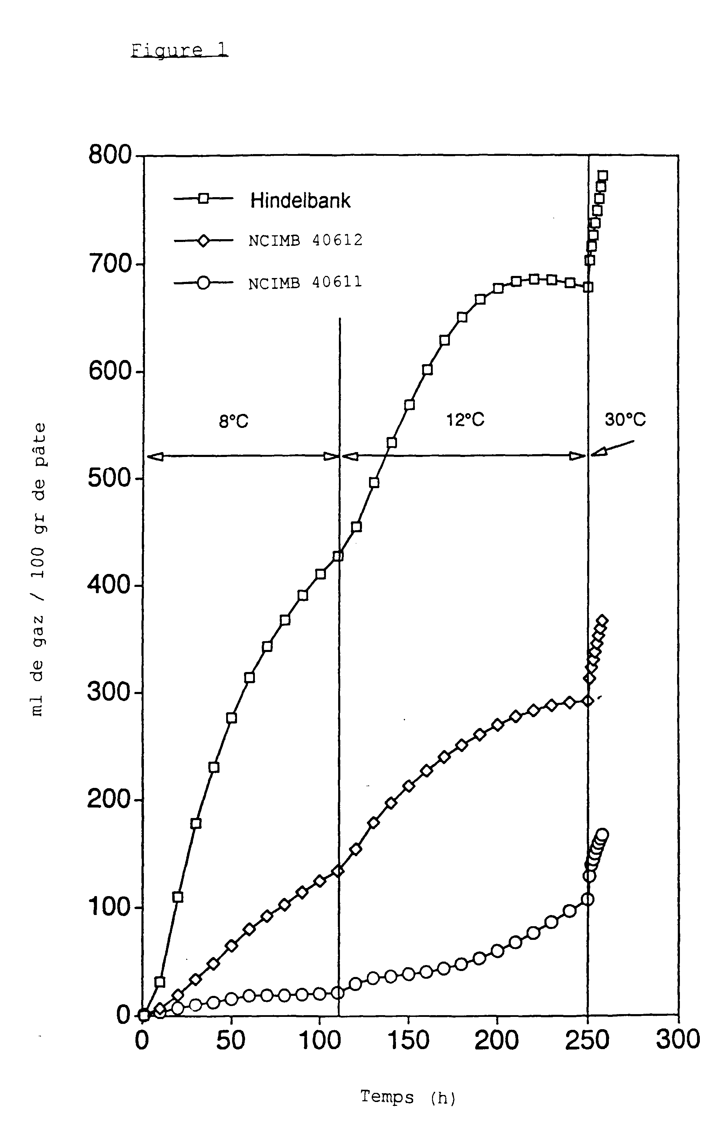

Cette souche présente une propriété Lti remarquable. Comme on peut le voir dans la figure 1 qui illustre les résultats obtenus selon le test "production de CO2 dans une pâte", cette souche est pratiquement inactive entre 3 et 12°C. Elle présente en effet une production de CO2 inférieure à 3 ml/h/kg de pâte à une température de 3-12°C. On constate également que le niveau de production de CO2 à 30°C monte rapidement.

De plus, les résultats obtenus par le test "production de CO2 sous un gradient de température", montre que cette souche ne dégage pas de gaz jusqu'à 18°C, après 12 jours de culture en milieu maltosé.

En outre, cette souche présente un taux de croissance exponentielle égal à 66% de celui de la levure LBB, et un rendement de croissance en processus fed-batch, selon le test décrit précedemment, équivalent à celui qui est déterminé de la même manière pour la souche LBB, soit environ 0,5 g de biomasse sèche/g de sucre.

Enfin, on ne trouve pas de révertants selon le test de stabilité génétique décrit plus haut. Cette souche est ainsi particulièrement stable.

### Exemple 2

On part d'une souche haploïde de Saccharomyces cerevisiae présentant une propriété Lti, telle que la souche NCIMB 40613 présentant le génotype MATa, mal3, lts500 que l'on a déposée selon le traité de Budapest le 28.01.94, à la National Collection of Industrial and Marine Bacteria Ltd. (NCIMB), P.O.Box 31, 135 Abbey Road, ABERDEEN AB9 8DG, Ecosse (Royaume uni).

On croise cette souche avec une souche haploïde Saccharomyces cerevisiae comprenant un allèle MAL actif mais sous répression catabolique et constitutif, notamment la souche NCIMB 40615 présentant le génotype MATa, leu2, trp1, ura3, MAL2-8c, MAL3, déposée selon le traité de Budapest le 28.01.94.

On fait sporuler les diploïdes, puis on croise les ségrégants et on sélectionne des souches de la même façon que dans l'exemple 1. Les pourcentages de souches ayant une propriété Lti et un potentiel de croissance donnés sont similaires à ceux obtenus dans l'exemple 1.

Parmi diverses souches ainsi obtenues, on a déposé, à titre d'exemple, la souche NCIMB 40612 mentionnée plus haut.

Comme on peut le voir dans la figure 1 qui illustre les résultats obtenus selon le test "production de CO2 dans une pâte", cette souche est pratiquement inactive entre 3 et 12°C. Elle présente en effet une production de CO2 inférieure à 7 ml/h/kg de pâte à une température de 3-8°C, et inférieure à 12 ml/h/kg de pâte à au moins 12°C, et on constate également que le niveau de production de CO2 à 30°C monte rapidement.

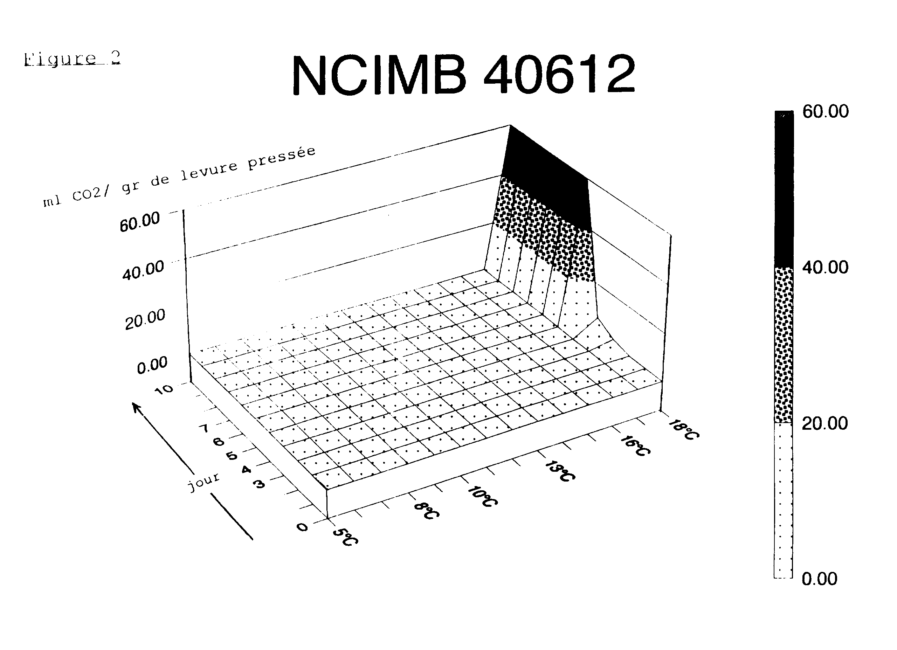

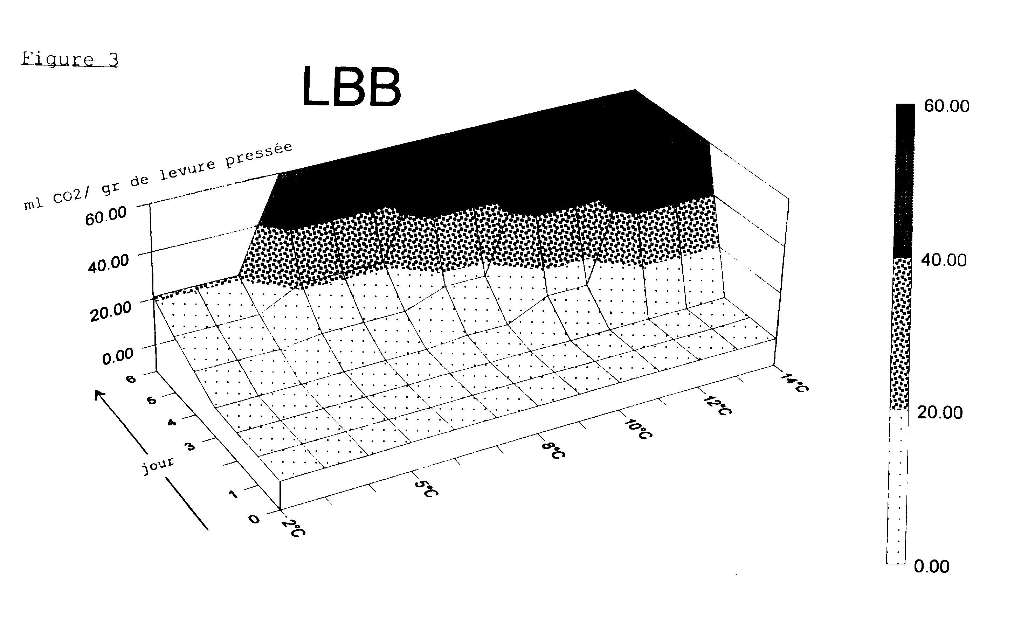

De plus, comme on peut le voir dans la figure 2 qui illustre les résultats obtenus par le test "production de CO2 sous un gradient de température", cette souche présente en outre une production de CO2 inférieure à 10 ml par g de levure pressée jusqu'à 18°C, après 4 jours de culture en milieu maltosé. Pour comparaison, on a également réalisé ce test pour la souche de référence LBB (voir la figure 3).

En outre, cette souche présente un taux de croissance exponentielle égal à 90% de celui de la levure LBB, et un rendement de croissance en processus fed-batch, selon le test décrit précédemment, équivalent à celui déterminé de la même manière pour la souche LBB, soit 0,5 g de biomasse sèche/g de milieu.

Enfin, on ne trouve pas de révertants selon le test de stabilité génétique décrit plus haut. Cette souche est ainsi également particulièrement stable.

### Exemple 3

On sèche les levures de boulangerie Lti afin de pouvoir les conserver pendant longtemps sous forme d'une poudre, de la manière suivante. On utilise une culture de la souche NCIMB 40612 centrifugée et lavée comprenant environ 20% de matière sèche. On sèche ensuite la levure d'une manière traditionnelle, par exemple comme décrit dans le brevet US 4358250. On obtient ainsi une poudre de levure sèche comprenant 96% de matière sèche.

On réhydrate ensuite ces levures de la manière suivante. On ajoute 10% de levure sèche à 90% d'eau à 30°C, puis on mélange doucement pendant 20 minutes, et enfin on ajoute une quantité d'eau à 4°C de manière à obtenir une suspension de levure ayant un taux de matière sèche désiré.

On peut noter que la levure déshydratée puis réhydratée conserve complètement son activité fermentaire.

### Exemple 4

On prépare une pâte de boulangerie réfrigérée et emballée comprenant la souche de levure NCIMB 40612 pressée. Pour cela, on utilise une culture fraîche pressée de levure NCIMB 40612, comme décrit précedemment dans le test "rendement en culture fed-batch".

On mélange alors une quantité de ces levures pressées (comprenant environ 35% de matière sèche) à de la farine, de l'eau, de la matière grasse et du sel de manière à avoir une pâte comprenant 0,2% en matière sèche de levure pressée, 60% de farine, 30% d'eau, 8% de margarine et 1,8% NaCl.

On emballe ensuite cette pâte de la même manière que celle décrite dans le brevet EP 0158590, à la différence que le plastique d'emballage qui est étanche à l'air comprend une soupape de sortie de gaz, c'est-à-dire une soupape qui permet la sortie de gaz de l'intérieur de l'emballage vers l'extérieur et qui interdit l'entrée d'air de l'extérieur vers l'intérieur de l'emballage. De plus, l'atmosphère interne de cet emballage comprend également 50% d'azote et 50% de dioxyde de carbone.

### Exemple 5

On prépare une pâte de boulangerie réfrigérée et emballée comprenant la souche de levure NCIMB 40611 pressée. Pour cela, on utilise une culture fraîche pressée de levure NCIMB 40611, comme décrit précedemment dans le test "rendement en culture fed-batch". Une quantité de la culture de levure pressée est alors mélangée à de la farine, de l'eau, de la graisse et du sel dans des proportions identiques à celles de l'exemple 4, puis la pâte est emballée de la même manière que celle décrite à l'exemple 4.

### Exemple 6

On prépare une pâte de boulangerie réfrigérée et emballée comprenant la souche de levure NCIMB 40612 réhydratée.

On utilise la suspension de levures NCIMB 40612 réhydratées de l'exemple 3 comprenant 10% de matière sèche, et on prépare ainsi une pâte comprenant 0,3% en matière sèche de levures réhydratées, 60% de farine, 30% d'eau, 8% de margarine et 1,7% NaCl. Puis, on emballe la pâte de la même manière que dans l'exemple 4.

### Exemple 7

On utilise la pâte de l'exemple 4 qui a été conservée à une température de réfrigération pendant 1 jour, puis on cuit directement la pâte à 220°C, pendant environ 15 minutes.

De même, on cuit de la même manière que ci-dessus les pâtes de l'exemple 4 conservées respectivement sous réfrigération, 7, 28 et 60 jours.

Enfin, pour comparaison on prépare une pâte ayant la même composition que celle décrite dans l'exemple 4, à la différence près que l'on ajoute des levures de boulangerie LBB (Hirondelle, Lesaffre S.A. France) à la place des levures Lti. On fait lever la pâte pendant 45 minutes à une température ambiante, puis on cuit la pâte dans les mêmes conditions que celles décrites ci-dessus.

Les trois pâtes cuites qui ont été conservées 7, 28 et 60 jours présentent un goût et une texture très proches de celles d'une pâte qui a été levée par des levures de la manière traditionnelle ci-dessus.

Par contre, la pâte cuite qui a été conservée 1 jour présente un goût et une texture un peu différentes de celles d'une pâte qui a été levée par des levures d'une manière traditionnelle. Ceci peut s'expliquer par le fait que lors du premier jour de conservation les levures NCIMB 40612 n'ont pas encore produit assez d'arômes et de gaz pour que la pâte soit très proche d'une pâte traditionnelle.

### Exemple 8

La pâte de l'exemple 5 qui a été conservée respectivement à une température de réfrigération pendant 2, 3, 7, 28 et 60 jours, est cuite directement à 220°C, pendant environ 15 minutes.

Les quatres pâtes cuites qui ont été conservées 3, 7, 28 et 60 jours présentent un goût et une texture très proches de celles d'une pâte qui a été levée par des levures de la manière traditionnelle ci-dessus.

Par contre, la pâte cuite qui a été conservée 2 jours présente un goût et une texture un peu différentes de celles d'une pâte qui a été levée par des levures LBB. Ceci peut s'expliquer par le fait que lors des deux premiers jour de conservation les levures NCIMB 40611 n'ont pas encore produit assez d'arômes et de gaz pour que la pâte soit très proche d'une pâte traditionnelle.

## Revendications

1. Procédé de construction d'une souche de levure de boulangerie présentant un phénotype Lti ("Low temperature inactive"), c'est à dire la capacité d'être pratiquement inactive mais de survivre dans une pâte à une température inférieure ou égale à 14°C à laquelle la pâte ne congèle pas, et d'être pratiquement inactive jusqu'à 18°C dans un milieu maltosé, dans lequel on croise premièrement une souche Saccharomyces cerevisiae haploïde présentant la capacité d'être inactive à une température de 3 à 10°C, de préférence au delà de 10°C, avec une souche Saccharomyces cerevisiae haploïde présentant au moins un allèle MAL actif mais sous répression catabolique, puis dans une deuxième étape on croise les ségrégants obtenus après sporulation, et enfin on sélectionne une souche diploïde prototrophe présentant un phénotype Lti en la soumettant à un test de production de CO₂ dans une pâte de boulangerie durant plusieurs jours à des températures comprises entre 3 et 30°C, un phénotype Mal actif mais sous répression catabolique, et un potentiel de croissance en processus fed-batch.

2. Procédé selon la revendication 1, caractérisé par le fait que dans la deuxième étape on croise des ségrégants qui sont chacun issu d'un croisement premier différent.

3. Procédé selon la revendication 1, caractérisé par le fait que l'allèle MAL actif mais sous répression catabolique peut être constitutif ou inductible dans la levure.

4. Procédé selon la revendication 1, caractérisé par le fait que pour vérifier le potentiel de croissance en processus fed-batch de ladite souche diploïde à sélectionner, on la développe dans un milieu de culture qui demande une métabolisation respiratoire de la ou des source(s) de carbone.

5. Souche de levure de boulangerie industrielle Saccharomyces cerevsiae susceptible d'être obtenu par le procédé selon la revendication 1, présentant un rendement de croissance en processus fed-batch de 0,1 à 0,5 g de biomasse sèche par g de sucre, une production de CO₂ inférieure à 15 ml/h/kg de pâte jusqu'à 8°C, inférieure à 20 ml/h/kg de pâte jusqu'à 12°C, et inférieure à 10 ml par g de levure pressée jusqu'à une température de 18°C après 4 jours de culture en milieu maltosé.

6. Souche selon la revendication 5, caractérisée en ce qu'elle présente un taux de croissance exponentielle dans un milieu qui demande une métabolisation respiratoire de la ou des source(s) de carbone, égal ou supérieur à 50% de celui d'une levure de boulangerie industrielle ne présentant pas une propriété Lti.

7. Souche selon la revendication 5, caractérisée en ce qu'elle présente un rendement de croissance en processus fed-batch supérieur à 0,5 g de biomasse sèche par g de sucre(s).

8. Souche selon les revendications 5 ou 7, caractérisée en ce qu'elle présente une production de CO₂ inférieure à 3 ml/h/kg de pâte jusqu'à 12°C.

9. Souche selon la revendication 5, caractérisée en ce qu'elle présente au moins un allèle MAL actif mais sous répréssion catabolique.

10. Souche de levure de boulangerie industrielle choisie dans le groupe formé par les souches de Saccharomyces cerevisiae NCIMB 40612 selon la revendication 5 et NCIMB 40611 selon les revendications 5 et 8.

11. Utilisation d'une souche de levure de boulangerie industrielle selon l'une des revendications 5 à 10 dans la production d'articles de boulangerie à cuire au four après conservation sous réfrigération.

12. Procédé de préparation d'une pâte de boulangerie réfrigérée et emballée, dans lequel on prépare une pâte en mélangeant une levure selon l'une des revendication 5 à 10 à au moins de l'eau et de la farine, puis on emballe la pâte dans un récipient qui comprend une soupape de sortie de gaz.

13. Procédé de préparation selon la revendication 12, caractérisé en ce que l'on mélange puis on emballe la pâte à des températures de réfrigération.

14. Procédé de préparation selon la revendication 12, caractérisé en ce que l'on emballe la pâte sous une atmosphère comprenant de l'azote ou du dioxyde de carbone ou un mélange des deux.

15. procédé de préparation selon la revendication 12, caractérisé en ce que l'on mélange la levure à la pâte à raison de 0,1 à 1% en matière sèche de levure pressée ou réhydratée.

16. Procédé de préparation selon la revendication 12, caractérisé par le fait que les levures contiennent du tréhalose.

17. Procédé de préparation selon la revendication 12, caractérisé en ce que l'on ajoute à la pâte 1,2 à 2% de NaCl.

18. Pâte de boulangerie comprenant au moins de l'eau, de la farine et une levure de boulangerie selon l'une des revendications 5 à 10, qui est conservée au moins 1 à 2 jours sous réfrigération.

## Patentansprüche

1. Verfahren zur Entwicklung eines Backhefestamms, der einen LTI-Phänotyp ("Low temperature inactive") zeigt, d.h. die Eigenschaft, in einem Teig bei einer Temperatur von 14°C oder weniger, bei der der Teig nicht gefriert, praktisch inaktiv zu sein, aber lebensfähig zu bleiben und in einem Maltosemilieu bis 18°C praktisch inaktiv zu bleiben, in dem man zunächst einen haploiden Stamm von Saccharomyces cerevisiae, der die Fähigkeit aufweist, bei einer Temperatur von 3 bis 10°C, vorzugsweise über 10°C, inaktiv zu sein, mit einem haploiden Stamm von Saccharomyces cerevisiae kreuzt, der mindestens ein aktives Allel MAL, das jedoch einer Katabolit-Repression unterliegt, aufweist, und dann in einem zweiten Schritt die nach Sporulation erhaltenen Segreganten kreuzt und schließlich einen diploiden prototrophen Stamm selektioniert, der einen LTI-Phänotyp zeigt, wenn man ihn während mehrerer Tage bei Temperaturen von 3 bis 30°C einem Test der CO₂-Erzeugung in einem Backteig unterwirft, und einen aktiven MAL-Phänotyp, jedoch unter Katabolit-Repression, und eine Wachstumsfähigkeit im Fed-batch-Verfahren aufweist.

2. Verfahren nach Anspruch 1, dadurch gekennzeichnet, daß man im zweiten Schritt Segreganten kreuzt, die jeweils aus einer anderen ersten Kreuzung hervorgegangen sind.

3. Verfahren nach Anspruch 1, dadurch gekennzeichnet, daß das aktive Allel MAL, jedoch unter Katabolit-Repression, in der Hefe konstitutiv oder induzierbar sein kann.

4. Verfahren nach Anspruch 1, dadurch gekennzeichnet, daß man zur Feststellung der Wachstumsfähigkeit dieses zu selektionierenden haploiden Stamms im Fed-batch-Verfahren diesen in einem Kulturmedium entwickelt, das eine Atmungsmetabolisierung der Kohlenstoffquelle(n) verlangt.

5. In dem Verfahren nach Anspruch 1 herstellbarer Industriebackhefestamm von Saccharomyces cerevisiae, der eine Wachstumsausbeute im Fed-batch-Verfahren von 0,1 bis 0,5 g Trockenbiomasse pro g Zucker, eine CO₂-Produktion von weniger als 15 ml/h/kg Teig bis zu 8°C, weniger als 20 ml/h/kg Teig bis 12°C und weniger als 10 ml pro g Presshefe bis zu einer Temperatur von 18°C nach 4 Tagen Kultur in Maltosemedium aufweist.

6. Stamm nach Anspruch 5, dadurch gekennzeichnet, daß er eine exponentielle Wachstumsrate in einem Medium, das eine Atmungsmetabolisierung der Kohlenstoffquelle(n) verlangt, aufweist, die gleich oder höher als 50 % der einer keine LTI-Eigenschaft aufweisenden Industriebackhefe ist.

7. Stamm nach Anspruch 5, dadurch gekennzeichnet, daß er eine Wachstumsausbeute im Fed-batch-Verfahren von über 0,5 g Trockenbiomasse pro g Zucker aufweist.

8. Stamm nach den Ansprüchen 5 oder 7, dadurch gekennzeichnet, daß er bis 12°C eine CO₂-Produktion von weniger als 3 ml/h/kg Teig aufweist.

9. Stamm nach Anspruch 5, dadurch gekennzeichnet, daß er mindestens ein aktives Allel MAL, jedoch unter Katabolit-Repression, besitzt.

10. Industriebackhefestamm, der aus der von den Stämmen Saccharomyces cerevisiae NCIMB 40512 nach Anspruch 5 und NCIMB 40611 nach den Ansprüche 5 und 8 gebildeten Gruppe ausgewählt ist.

11. Verwendung eines Industriebackhefestamms nach einem der Ansprüche 5 bis 10 in der Herstellung von Backwaren, die nach Konservierung unter Kühlung im Ofen zu backen sind.

12. Verfahren zur Herstellung eines gekühlten und verpackten Backteigs, bei dem man einen Teig herstellt, indem man eine Hefe nach einem der Ansprüche 5 bis 10 mindestens mit Wasser und Mehl mischt und den Teig dann in einem Behälter verpackt, der ein Gasaustrittsventil aufweist.

13. Herstellungsverfahren nach Anspruch 12, dadurch gekennzeichnet, daß man den Teig bei Kühltemperaturen mischt und dann verpackt.

14. Herstellungsverfahren nach Anspruch 12, dadurch gekennzeichnet, daß der Teig unter einer Atmosphäre verpackt wird, die Stickstoff oder Kohlendioxid oder eine Mischung dieser beiden enthält.

15. Herstellungsverfahren nach Anspruch 12, dadurch gekennzeichnet, daß man die Hefe mit dem Teig in einer Menge von 0,1 bis 1 % in Trockenmasse Presshefe oder rehydratisierter Hefe mischt.

16. Herstellungsverfahren nach Anspruch 12, dadurch gekennzeichnet, daß die Hefen Trehalose enthalten.

17. Herstellungsverfahren nach Anspruch 12, dadurch gekennzeichnet, daß man dem Teig 1,2 bis 2 % NaCl beigibt.

18. Backteig, der mindestens Wasser, Mehl und Backhefe nach einem der Ansprüche 5 bis 10 enthält und mindestens 1 bis 2 Tage unter Kühlen konserviert ist.

## Claims

1. Procedure for constructing a strain of baker's yeast having an LTi (low temperature inactive) phenotype, namely the capacity to be practically inactive but to survive in a dough at a temperature of less than or equal to 14°C at which the dough does not freeze, and to be practically inactive up to 18°C in a maltose medium, wherein first of all a haploid Saccharomyces cerevisiae strain having the capacity of being inactive at a temperature of 3 to 10°C, preferably in excess of 10°C, is crossed with a haploid Saccharomyces cerevisiae strain having at least one MAL allele which is active but under catabolic repression, and then in a second stage the segregants obtained after sporulation are crossed, and finally a prototrophic diploid strain is selected having an LTi phenotype by subjecting it to a test for CO₂ production in a baker's dough for several days at temperatures between 3 and 30°C, an MAL phenotype, which is active but is under catabolic repression, and which has a potential for growth in a fed-batch process.

2. Process according to claim 1, characterized in that in the second stage the segregants are crossed, each resulting from a first different cross.

3. Process according to claim 1, characterized in that the MAL allele which is active but is under catabolic repression can be a constituent of or can be induced in the yeast.

4. Process according to claim 1, characterized in that in order to verify the growth potential in a fed-batch process of the said diploid strain to be selected, it is developed in a culture medium which requires respiratory metabolism of the carbon source or sources.

5. Strain of industrial baker's yeast Saccharomyces cerevisiae capable of being obtained by the process according to claim 1, having a growth yield in a fed-batch process of 0.1 to 0.5 g of dry biomass per gram of sugar, a CO₂ production of less than 15 ml/h/kg of dough up to 8°C, less than 20 ml/h/kg of dough up to 12°C, and less than 10 ml per gram of pressed yeast up to a temperature of 18°C after 4 days of culture in a maltose medium.

6. Strain according to claim 5, characterized in that it has an exponential rate of growth in a medium which requires respiratory metabolism of the source or sources of carbon, equal to or greater than 50 % of that of an industrial baker's yeast not having an LTi property.

7. Strain according to claim 5, characterized in that it has a growth yield in a fed-batch process greater than 0.5 g of dry biomass per gram of sugar(s).

8. Strain according to claims 5 or 7, characterized in that it has a CO₂ production of less than 3 ml/h/kg of dough up to 12°C.

9. Strain according to claim 5, characterized in that it has at least one MAL allele which is active but is under catabolic repression.

10. Strain of industrial bakers yeast selected from the group formed of strains of Saccharomyces cerevisiae NCIMB 40612 according to claim 5 and NCIMB 40611 according to claims 5 and 8.

11. Use of a strain of industrial baker's yeast according to one of claims 5 to 10 in the production of articles of bakery to be baked in an oven after storage under refrigeration.

12. Process for preparing a refrigerated and packaged bakers dough, in which a dough is prepared by blending a yeast according to one of claims 5 to 10 with at least water and flour, and the dough is then packaged in a container which comprises a gas outlet valve.

13. Preparation process according to claim 12, characterized in that the dough is blended and then packaged at refrigeration temperatures.

14. Preparation process according to claim 12, characterized in that the dough is packaged under an atmosphere comprising nitrogen or carbon dioxide or a mixture of the two.

15. Preparation process according to claim 12, characterized in that yeast is blended with dough at a rate of 0.1 to 1% of dry matter of pressed or rehydrated yeast.

16. Preparation process according to claim 12, characterized in that the yeasts contain trehalose.

17. Preparation process according to claim 12, characterized in that 1.2 to 2% of NaCl is added to the dough.

18. Bakers dough comprising at least water, flour and a bakers yeast according to one of claims 5 to 10, which is stored at least 1 to 2 days under refrigeration.
